# EUROPEAN PATENT SPECIFICATION

(11) **EP 3 030 150 B1**
(45) Date of publication and mention of the grant of the patent: **21.06.2023**
(21) Application number: 14834640.6
(22) Date of filing: 07.08.2014
(51) Int. Cl.: A61B 5/145, A61B 5/00

(54) **ANALYTE SENSOR TRANSCEIVER CONFIGURED TO PROVIDE TACTILE, AND OPTIONALLY VISUAL AND / OR AURAL, FEEDBACK**
ZUR BEREITSTELLUNG VON TAKTILEM, UND GEGEBENENFALLS VISUELLEM UND/ODER AKUSTISCHEM, FEEDBACK KONFIGURIERTER ANALYTSENSOR-SENDEEMPFÄNGER
ÉMETTEUR-RÉCEPTEUR À CAPTEUR D'ANALYTE CONFIGURÉ POUR FOURNIR UNE RÉTROACTION TACTILE ET ÉVENTUELLEMENT VISUELLE ET/OU SONORE

(30) Priority: 09.08.2013 US 201361864174 P; 13.08.2013 US 201361865373 P; 24.09.2013 US 201361881679 P
(43) Date of publication of application: 15.06.2016
(60) Divisional application: 23168079.4
(73) Proprietor: Senseonics, Incorporated, Germantown, MD 20876-7005 (US)
(72) Inventor: TANKIEWICZ, Szymon, Germantown, MD 20876 (US); EMKEN, Jeremy, Germantown, MD 20876 (US); DEHENNIS, Andrew, Germantown, MD 20876 (US); WHITEHURST, Todd, Germantown, MD 20876 (US)
(74) Representative: Elkington and Fife LLP
(86) International application number: PCT/US2014/050141
(87) International publication number: WO 2015/021273

(56) References cited:
- WO-A1-2011/091336
- WO-A1-2013/055962
- WO-A2-2011/003035
- US-A1- 2007 163 880
- US-A1- 2011 050 428
- US-A1- 2012 223 251
- US-A1- 2012 245 444
- US-B1- 6 422 066

## Description

### CROSS-REFERENCE TO RELATED APPLICATIONS

The present application claims the benefit of priority to U.S. Provisional Application Serial No. 61/864,174, filed on August 9, 2013; U.S. Provisional Application Serial No. 61/865,373, filed on August 13, 2013; and U.S. Provisional Application Serial No. 61/881,679, filed on September 24, 2013.

### FIELD OF THE INVENTION

The invention is directed to an on-body, wearable transceiver for communication with an analyte sensor, where the sensor is implanted subcutaneously, intravenously, or intraperitoneally. The transceiver may provide visual, vibratory/tactile, and/or aural notifications/feedback to the user. The transceiver may also provide wireless communication to and exchange data with a smartphone or other device, which may also have visual, vibratory/tactile, and/or aural notifications/feedback.

### BACKGROUND OF INVENTION

Conventional continuous analyte (e.g., glucose) monitoring systems include a sensor, a transceiver worn on the user and configured for communication with the sensor, and a receiver, separate from the transceiver and configured to receive data transmissions from the transceiver and to provide notifications and feedback to the user. In conventional systems, all alarms and/or other aural, visual or vibratory notifications are provided by the receiver. The receiver shows the user both passive and actionable information and provides visual/aural/vibratory alarms. In some systems, the transceiver is also connected (either by wire or wirelessly) to either to an insulin pump or a personal communication device, such as a smartphone, in which case the visual and/or aural and/or vibratory alarm/notification systems are incorporated into either the insulin pump or the communication device.

A disadvantage of conventional analyte monitoring systems is that in order for a user to receive any visual, aural or vibratory alarms and/or information related to the analyte measurements, the user is required to be within close proximity of the receiver in order to see, hear, or feel the alarm. Thus, improvements in analyte monitoring systems are desired.

WO 2013/055962 discloses a wireless power and data exchange between an external reader and an implanted sensor. WO 2011/091336 discloses a method and apparatus for providing notification in analyte monitoring systems.

### SUMMARY OF THE INVENTION

An analyte monitoring system embodying aspects of the present invention comprises an analyte sensor and a transceiver which may be externally worn, wherein the transceiver is configured for communication with the sensor and includes the capabilities of having the transceiver vibrate and optionally generate one of an aural or visual signal to prompt the user about sensor and/or transceiver information, in addition to optionally having these capabilities reside on the smartphone application.

One aspect of the present invention provides a transceiver according to claim 1, for interfacing with an analyte sensor.

The interface device is an antenna configured to wirelessly convey the power signal to an antenna of the analyte sensor and to wirelessly receive the data signals from the antenna of the analyte sensor. In some embodiments, the notification device may be configured to generate one or more of a vibrational, aural, and visual signal if the transceiver is placed for optimal signal exchange between the analyte sensor and the transceiver.

The interface device of the transceiver receives analyte data from the analyte sensor, and the transceiver comprises a processor configured to calculate an analyte concentration value based on the received analyte data. In some embodiments, the transceiver may include a wireless communication circuit configured to wirelessly transmit the analyte concentration to a smartphone or other device.

In some embodiments, the notification device may be configured to emit an aural alarm when the calculated analyte concentration value exceeds or falls below a threshold value. The notification device includes a vibration motor configured to generate a vibrational signal when the calculated analyte concentration value exceeds or falls below a threshold value. In some embodiments, the notification device may include one or more LEDs configured to generate one or more visual signals.

The transceiver includes a battery, and the notification device is configured to generate a vibrational signal and optionally one or both of an aural and visual signal if the power level of the battery falls below a threshold. The transceiver is an on-body, wearable transceiver.

Another aspect of the present invention provides a system for detecting an amount or concentration of an analyte in vivo within a living organism, according to claim 6.

### BRIEF DESCRIPTION OF THE DRAWINGS

The accompanying drawings, which are incorporated herein and form part of the specification, illustrate various, non-limiting embodiments of the present invention. In the drawings, like reference numbers indicate identical or functionally similar elements.
FIG. 1 is cross-sectional, perspective view of a transceiver embodying aspects of the invention.
FIG. 2 is an exploded, perspective view of a transceiver embodying aspects of the invention.
FIG. 3 is a schematic view of an analyte monitoring system embodying aspects of the invention.

### DETAILED DESCRIPTION OF THE INVENTION

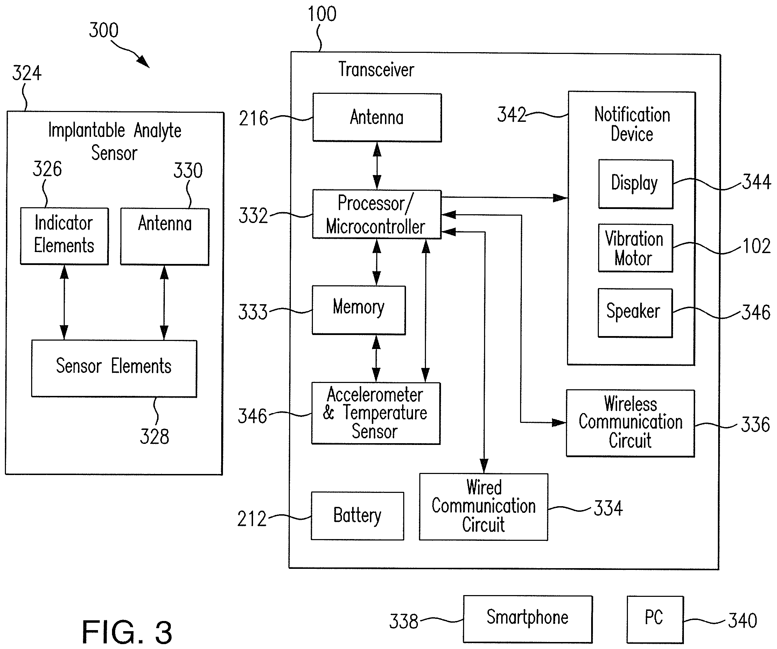

FIG. 3 is a schematic view of an analyte monitoring system 300 embodying aspects of the present invention. The analyte monitoring system 300 may include a sensor 324 and an external transceiver 100 configured for communication with the sensor 324. In the embodiment shown in FIG. 3, the sensor 324 may be an implantable sensor configured to be implanted in a living animal (e.g., a living human). In some non-limiting embodiments, the sensor 324 may be an implantable sensor configured to be implanted, for example, in a living animal's arm, wrist, leg, abdomen, peritoneum, or other region of the living animal suitable for sensor implantation. For example, in one non-limiting embodiment, the sensor 324 may be implanted beneath the skin (e.g., in the subcutaneous tissues). In some embodiments, the sensor 324 may be an analyte sensor configured to detect an analyte (e.g., glucose or oxygen) in a patient's interstitial fluid, blood, or intraperitoneal fluid. In some embodiments, as illustrated in FIG. 3, the sensor 324 may include indicator elements 326 and sensor elements 328 to detect analyte data and convey/communicate the analyte data to the external transceiver 100.

The transceiver 100 is an on-body/wearable device. For example, in some embodiments, the transceiver 100 may be held in place by a band (e.g., an armband, wristband, leg band, or belt) and/or adhesive, and the transceiver 100 may convey (e.g., periodically, such as every two minutes, and/or upon user initiation) measurement commands (i.e., requests for measurement information) to the sensor 324.

The transceiver 100 receives analyte data from the sensor 324 and calculate an analyte concentration based on the received analyte data. The transceiver 100 includes a processor 332 that performs the analyte concentration calculation. In some non-limiting embodiments, the analyte concentration calculation may include one or more features described in U.S. Patent Application Publication No. 2014/001 8644. The transceiver 100 may also include memory 333 (e.g., Flash memory), which may be non-volatile and/or capable of being electronically erased and/or rewritten. The memory 333 may store analyte data received from the sensor 324 and/or calculated analyte concentrations.

The sensor 324 includes an antenna 330, and the sensor 324 wirelessly conveys the analyte data to the external transceiver 100. The transceiver 100 includes an antenna 216 that is capable of coupling with the antenna 330 of the sensor 324. The antenna 216 wirelessly receives analyte data and optionally other data (e.g., temperature data) from the sensor 324 and wirelessly conveys power and optionally data (e.g., commands) to the sensor 324. However, in general, it is not required that the sensor 324 and transceiver 100 have antennas, and, in alternative examples (not presently claimed), the system 300 may include a transdermal needle tip that provides a wired connection between the external transceiver 100 and the sensor 324. In these examples, the sensor 324 may convey the analyte data to the transceiver 100 via the wired connection provided by the transdermal needle tip, and the transceiver 100 may convey power and/or data (e.g., commands) to the sensor 324 via the wired connection provided by the transdermal needle tip.

In some embodiments, the transceiver 100 may have a wired communication circuit 334 that enables wired connection to an external device, such as a smartphone 338 or personal computer (PC) 340. In some non-limiting embodiments, the wired communication circuit 334 may include a connector, such as, for example, a Micro-Universal Serial Bus (USB) connector. However, in alternative embodiments, other connectors may be used. The transceiver 100 may exchange data to and from the external device through the wired communication circuit 334 and/or may receive power through the wired communication circuit 334.

In some embodiments, the transceiver 100 may wirelessly transmit the calculated analyte concentration to a smartphone 338 or other external device. In a non-limiting embodiment, the transceiver 100 may include a wireless communication circuit 336 to wirelessly transmit the analyte concentration. The wireless communication circuit 336 may employ one or more wireless communication standards to wirelessly transmit the data. The wireless communication standard(s) employed may include any suitable wireless communication standard, such as, for example, an ANT standard, a Bluetooth standard, or a Bluetooth Low Energy (BLE) standard (e.g., BLE 4.0).

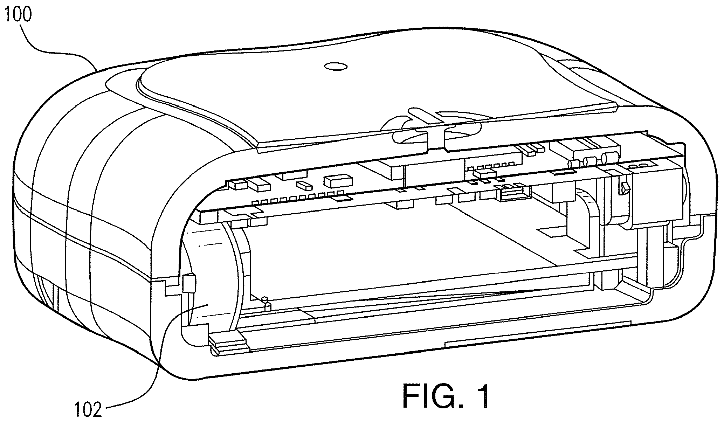

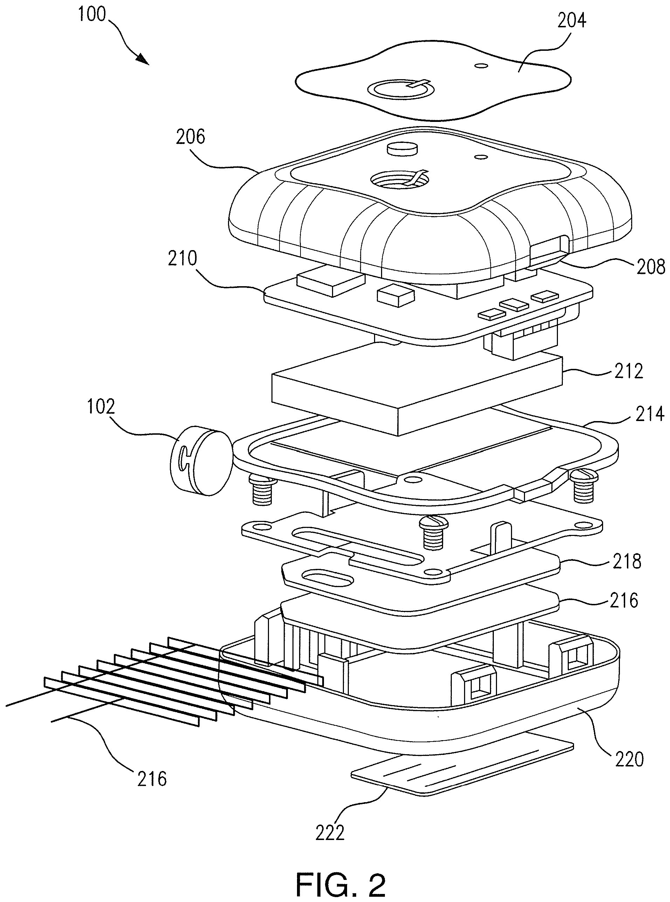

FIGS. 1 and 2 are cross-sectional and exploded views, respectively, of a non-limiting embodiment of the transceiver 100, which may be included in the analyte monitoring system 300. As illustrated in FIG. 2, in some non-limiting embodiments, the transceiver 100 may include a graphic overlay 204, front housing 206, button 208, printed circuit board (PCB) assembly 210, battery 212, gaskets 214, antenna 216, frame 218, back housing 220, ID label 222, and/or vibration motor 102. In a non-limiting embodiment, the transceiver electronics may be assembled using standard surface mount device (SMD) reflow and solder techniques. In one embodiment, the electronics and peripherals may be put into a snap together housing design in which the front housing 206 and back housing 220 may be snapped together. However, this is not required, and in some alternative embodiments, the front housing 206 and back housing 220 in another manner (e.g., ultrasonic welding). In some embodiments, the full assembly process may be performed at a single external electronics house. However, this is not required, and, in alternative embodiments, the transceiver 100 may be performed at one or more electronics houses, which may be internal, external, or a combination thereof. In some embodiments, the assembled transceiver may be programmed and functionally tested. In some embodiments, assembled transceivers 100 may be packaged into their final shipping containers and be ready for sale.

In some embodiments, the transceiver 100 may provide on-body alerts to the user in a visual, aural, and/or vibratory manner, regardless of proximity to a smartphone. As illustrated in FIG. 3, the transceiver 100 includes a notification device 342 that generates vibratory alerts and optionally visual and/or aural alerts. In addition to a vibration motor 102, the notification device 342 may include one or more of a display 344 and a speaker 346, which respectively generate the vibratory, and optionally visual and/or aural. In some embodiments, the transceiver 100 may be configured to vibrate and/or generate an audio or visual signal to prompt the user about analyte readings outside an acceptable limit, such as hypo/hyper glycemic alerts and alarms in the case where the analyte is glucose.

The vibrational, visual, and/or aural tone feedback provided by the transceiver 100 can enable the use of different patterns/rhythms/melodies that have various meanings corresponding to the status of the transceiver 100 or the inserted sensor 324, or the analyte concentration. For example, in one non-limiting embodiment, the transceiver 100 might be calibrated to provide a long, repeatable vibration, with or without an aural or visual alarm, when a user's glucose concentration becomes too low or too high. In some embodiments, a vibration motor 102 of the transceiver 100 may communicate various messages/alerts to the user through Morse code like patterning and sequencing (e.g., long-long-short-short) and/or different vibration speeds and intensities. In a non-limiting embodiment, a circuit, such a supply voltage controller, may control the vibration speed and intensity. In some embodiments, different patterns of audio feedback, which may include different volumes, frequencies, time on-off (duty cycle), melodies, and/or rhythms may be used to communicate various messages/alerts to the user. The vibration motor could also be set via an interface to wake up the subject with various vibration patterns.

In some embodiments, the display 344 may be any suitable display such as, for example and without limitation, a display that includes one or more light emitting diodes (LEDs), one or more liquid crystal displays (LCDs), and/or one or more organic light emitting devices (OLEDs). In some non-limiting embodiments, the transceiver 100 might be calibrated to provide a visual alert (e.g., one or more LEDs or other light sources may turn on and off in a specific pattern and/or emit light of different intensities and/or frequencies/colors) when a user's glucose concentration becomes too low or too high. For example, in some non-limiting embodiments, the display 344 of the notification device 342 may be capable of displaying more than one color. For instance, in some non-limiting embodiments, the display 344 may include dual LED (e.g., yellow/green) or a tri-color LED (i.e., blue/yellow/green). A display 344 providing different colors may enhance communication modes by adding color as variable. For instance, by using more than one LED (e.g., the dual LED or the tri-color LED) or other light source, the display 344 may generate a blinking yellow-green-yellow-etc. visual signal and/or a long yellow-short yellow-short green-short green-etc. visual signal to communicate various messages/alerts to the user.

In a non-limiting embodiment, the combination of visual, aural, and/or vibratory patterns may communicate different messages/alerts than if the visual, aural, and/or vibratory patterns were communicated alone. In some embodiments, the transceiver 100 may provide certain patterns of a vibratory and/or aural and/or visual alert to prompt the user when a calibration point is needed or is going to be needed, and/or when the battery (e.g., battery 212) has low power (e.g., a power level below a threshold) and needs to be recharged. In some embodiments, the smartphone 338 or other device communicating with the transceiver may also have visual, aural, or vibratory alarms and notifications.

The vibrational, visual, and/or aural feedback of the transceiver 100 can also alert the user regarding the status of the telemetry system with the sensor 324. For example, for systems in which transceiver 100 delivers power to the sensor 324 (e.g., by radio frequency signals via an inductive antenna), the visual, aural, and/or vibratory feedback can prompt the subject regarding how well the two systems are coupled. In other embodiments, the vibrational and/or aural feedback of the transceiver 100 can assist the user in adjusting the relative position of the transceiver 100 and optimize coupling between the transceiver 100 and the sensor 324 without having visual feedback. That is, the user can adjust the position of the transceiver 100 worn under a piece of clothing (e.g., shirt, etc.) without looking at the transceiver 100 - the buzzer/vibrator signals of the transceiver 100 would indicate to the user if the sensor 324 is well within the range and if the readings are correct. Accordingly, in some embodiments, the transceiver 100 may be used to alert the user to an optimal location of the transceiver 100 over the implanted sensor (e.g., a location in which the transceiver 100 and sensor 324 are inductively coupled), which allows the user to adjust the transceiver 100.

Embodiments of the present invention also include novel ways for building in the vibration motor 102 to the transceiver PCB assembly 210 and housing 206 and 220. The assembly of the vibration motor 102 in the housing enables the coupling of force to the subject. In some embodiments, the axis of vibration may be perpendicular with the body contacting surface of the transceiver 100 to efficiently couple the force of the vibration to the subject. However, this is not required, and, in some alternative embodiments, the axis of vibration may be co-planar with the body contact surface. In some embodiments, the vibration motor 102 may be attached to the transceiver housing, frame, or other components within the transceiver 100. In some embodiments, attachment of the vibration motor 102 to the transceiver housing enables an aural alert out of the vibration motor 102, by creating an audible sound as the vibration motor 102 vibrates against the internal surface of the transceiver 100. The vibration motor 102 may be attached to a special membrane, which may amplify the audio aspect of the vibration and act as a speaker cone membrane. In other embodiments, the vibrational and/or aural feedback of the transceiver 100 can be combined with a variable vibration speed system by controlling the supply voltage to vibration motor 102. This would enable both tactile/audio feedback intensity to be varied and enhance communication modes.

In some embodiments, the transceiver 100 may include an accelerometer and temperature sensor unit 346. The accelerometer and temperature sensor unit 346 may include an accelerometer that monitors motion. In some non-limiting embodiments, the accelerometer may improve analyte monitoring by providing motion information that can be used to help determine movement-related artifacts or noise that may be present within the monitoring signal. In other embodiments, the transceiver 100 may additionally or alternatively use information from the accelerometer to provide feedback to the user of the device in order to aid the user in, for example, moving the sensor 324 to the correct position or orientation. In other embodiments, the accelerometer may be used for sleep monitoring. In some non-limiting embodiments, the analyte monitoring system may include different analyte pattern recognition parameters (e.g., different glycemic pattern recognition parameters) for different states, such as, for example, awake/active, awake/sedentary, and/or asleep. This further information may be translated into different alarm types for the subject. Furthermore, the transceiver 100 may have a settable alarm clock functionality, and the transceiver 100 may alert the user following sleep pattern and activity levels.

In some embodiments, the accelerometer may act as a button like interface. This would alleviate the need to have a mechanical button, or the button like interface of the accelerometer could be used in addition to one or more mechanical buttons.

In some embodiments, the transceiver 100 may notify a smartphone (e.g., smartphone 338) of an emergency condition. In some non-limiting embodiments, the transceiver 100 may detect an emergency condition if the accelerometer detects a period of low acceleration followed by high acceleration (e.g., a fall event). In some non-limiting embodiments, the transceiver 100 may detect an emergency condition if alarms are ignored (e.g., no receipt of user input) for a period of sustained hypoglycemia or hyperglycemia. In some embodiments, notifying the smartphone of an emergency condition may cause the cause the smartphone to contact (e.g., via email or text) one or more emergency contacts. In this manner, the transceiver 100 may enable automatic emergency contact for emergency conditions such as, for example, hypoglycemic shock.

The transceiver 100 may include functionality to act as the hub for body worn or local proximity sensors. These sensors could include, for example, oxygen levels or pulse oximeters, sweat detectors, heart rate monitors, and/or gyroscopes. Inclusion of one or more temperature sensors enable the ability to track core, subcutaneous and skin temperature, which could provide feedback on physiological states as well. These body worn or local proximity sensors could communicate using a wireless communication standard (e.g., BLE) or be directly wired to the transceiver 100. The transceiver 100 may log data received from the body worn or local proximity sensors and transmit the data (e.g., using a wireless communication standard) to a paired smartphone 338 or PC 340, cellular or Wi-Fi. In some embodiments, the transceiver 100 may have a direct interface to an insulin pump to control dosing of bolus or basal rates depending on glycemic or other measurements. In some non-limiting embodiments, the sensor hub may provide an interface for feedback of body movements and physiological signals (e.g., for use with a gaming system that interacts with movements of the human body).

In some embodiments, the transceiver 100 may have various forms of energy harvesting mechanisms to slowly charge the battery 212 in order to increase uninterrupted run time. For example, in one non-limiting embodiment, the transceiver 100 may include a solar panel on the transceiver enclosure (e.g., on front housing 206). For another example, the transceiver 100 may additionally or alternatively include one or more of thermo-, magneto-, piezo-, vibration, and/or movement mechanisms, etc. as a source of energy. In one non-limiting embodiment, the electromagnetic waves from cellular telephony, Bluetooth, GPS, Wi-Fi, or other frequency bands can be used for harvesting energy.

In some embodiments, the transceiver 100 may include splash-proof or waterproof features. This would enable the user to use the transceiver 100 according to appropriate splash-proof or waterproof guidelines. For example, in embodiments where the transceiver 100 is waterproof, a user could take as shower while wearing the waterproof transceiver 100.

In some embodiments, the transceiver 100 may include a secondary processor. In some embodiments, the secondary processor may be part of the wireless communication circuit 336 and may perform the functionality of communicating according to a wireless communication standard (e.g., BLE). In embodiments where the transceiver 100 includes a secondary processor, the secondary processor may help processor 332 handle some peripherals. For example, the secondary processor may help to handle interrupts from buttons, drive LEDs (e.g., in display 344), control vibration motor 102, buzzer or speaker 346, etc. This way transceiver 100 could potentially save energy by using the secondary processor instead of processor 332. Use of the secondary processor may also enable use of a smaller package for processor 332 with less pins. The secondary processor and processor 332 may be connected through an I/O internal interface exchanging data and commands. The secondary processor and processor 332 may wake up each other and perform certain tasks. This way, one or both of the secondary processor and processor 332 may "sleep" until activity is needed. In some embodiments, the secondary processor (e.g., when part of the wireless communication circuit) may be used to upgrade the firmware of processor 332 wirelessly so no wired/physical connection would be needed.

In some embodiments, the transceiver 100 may incorporate a headset (e.g., a BLE headset). In some non-limiting embodiments, the headset may be suitable for blind and/or visually impaired people. The headset may inform the user about analyte levels, alerts, coupling issues, reminders, etc. The headset may have microphone so the user can issue voice command to a smartphone 338 and/or transceiver 100. In some non-limiting embodiments, using the headset, the user may be able to issue voice commands such as, for example, measure analyte now, battery level, tell me the glucose trend, time, etc.. The headset may use the wireless communication circuit 336 of the transceiver 100 (e.g., internal transceiver BLE) for issuing voice commands, or the headset may request and use iPod/iPhone/smartphone/etc. for that purpose. However, in some alternative embodiments, the transceiver 100 may have an internal microphone and speaker so that a headset would not be needed, and the transceiver 100 may instead perform the functionally of the headset described above.

In some embodiments, the transceiver 100 may have a display 344. The display 344 may provide information about one or more of, for example, time, date, analyte values, trends, activities log, alerts/alarms, battery level, sensor signal, BLE signal, etc. In some non-limiting embodiments, the display 344 may be a color display and/or may be a high resolution display or an organic light emitting device (OLED) display. In some embodiments, all useful information (or at least vital information) may be available through the display 344 so that the patient does not have to rely on a smartphone (e.g., smartphone 338).

In some non-limiting embodiments, the transceiver 100 may have embedded (web) radio, video, and mp3 player capabilities. In some non-limiting embodiments, the display 344 may enable WWW, chat, social games, and services like competing with fellow users in calories/steps counts, etc. For example, the WWW capability may be achieved by using Wi-Fi enabled ICs.

The transceiver 100 has a battery 212. In some non-limiting embodiments, the battery 212 may be a disposable or rechargeable battery. In some non-limiting embodiments, the battery 212 may be a replaceable battery. With the ability to swap replaceable batteries, one transceiver 100 could be used with a plurality of swappable batteries for longer periods between charging, which would be useful during times when recharging is not possible (e.g., camping, traveling, etc.). In some non-limiting embodiments, transceiver 100 may be compatible with bigger, swappable batteries so the user could use transceiver 100 for longer time while accepting increased transceiver size.

In some embodiments, the accelerometer and temperature sensor unit 346 may include a temperature sensor (e.g., a thermistor, thermocouple, or resistance temperature detector). The temperature sensor may be used monitor body temperature. In some non-limiting embodiments, as illustrated in FIG. 3, the accelerometer and temperature sensor may be part of a single unit 346. However, this is not required, and, in some alternative embodiments, the accelerometer and temperature sensor may be provided as separate units, or the transceiver 100 may include only one of the accelerometer and the temperature sensor.

In some embodiments, the transceiver 100 may include a Global Positioning System (GPS) unit having the functionality to acquire a GPS signal. The GPS unit may implement hardware and/or software functionality that enables monitoring of motion. In some non-limiting embodiments, the GPS unit may improve glucose monitoring by providing motion information that can be used to help determine movement-related artifacts or noise that may be present within the monitoring signal. In some embodiments, the GPS unit may provide the transceiver 100 with the ability to communicate exact positions for patients that may go into hypoglycemic shock and would need emergency personal notified of their location for treatment. For example, the location of the patient may be communicated through the transceiver's wireless capabilities and/or through a cellular or Wi-Fi handset.

In some embodiments, the transceiver 100 may include an ambient light sensor (e.g., photodiodes, phototransistors, photoresistors or other photosensitive elements). In some non-limiting embodiments, the ambient light sensor may be used to predict potential ambient light issues. For instance, in embodiments where the sensor 324 is an optical sensor, ambient light could interfere with the sensor's analyte measurements, the ambient light sensor could be used to flag or indicate analyte measurements that may not be reliable due to ambient light (e.g., when ambient light exceeds one or more thresholds). In other embodiments, transceiver 100 may incorporate the measured intensity of ambient light into the calculation of analyte concentration.

Embodiments of the present invention have been fully described above with reference to the drawing figures. Although the invention has been described based upon these preferred embodiments, it would be apparent to those of skill in the art that certain modifications, variations, and alternative constructions could be made to the described embodiments within the scope of the appended claims. For example, circuitry of the sensor and transceiver may be implemented in hardware, software, or a combination of hardware and software. The software may be implemented as computer executable instructions that, when executed by a processor, cause the processor to perform one or more functions. For another example, although the vibration motor 102 is illustrated in FIGS. 1 and 2 as having a cylindrical shape, this is not required, and, in alternative embodiments, vibration motors having other shapes may be used.

## Claims

1. An on-body, wearable transceiver (100) for interfacing with an implantable analyte sensor (324) in a continuous analyte monitoring system, the transceiver comprising:
an interface device (216) configured to convey a power signal to the implantable analyte sensor and to receive data signals from the implantable analyte sensor;
a battery (212);
a processor (332) configured to calculate an analyte concentration value based on one or more of the received data signals from the implantable analyte sensor;
a notification device (342) including a vibration motor (102), wherein the notification device is configured to generate:
a vibrational signal having a first pattern to indicate that the calculated analyte concentration value has exceeded or has fallen below a threshold value, and
a vibrational signal having a second pattern different than the first pattern to indicate that a power level of the battery is below a threshold; and
a communication circuit (336) configured to transmit data to an external device (338, 340),
wherein the interface device is an antenna configured to wirelessly convey the power signal to an antenna (330) of the implantable analyte sensor and to wirelessly receive the data signals from the antenna of the implantable analyte sensor.

2. The transceiver of claim 1, wherein the notification device is configured to generate one or more of a vibrational, aural, and visual signal if the transceiver is placed for optimal signal exchange between the implantable analyte sensor and the transceiver.

3. The transceiver of claim 1, wherein the notification device comprises one or more LEDs configured to generate one or more visual signals.

4. The transceiver of claim 1, comprising at least one of the following features:
(i) an accelerometer;
(ii) a temperature sensor embedded in the accelerometer;
(iii) a Global Positioning System, GPS, unit configured to acquire a GPS signal and take location measurements; and
(iv) a temperature sensor.

5. The transceiver of claim 1, configured to be held in place on the living animal or human body by a band and/or adhesive.

6. A system (300) for detecting an amount or concentration of an analyte in vivo within a living organism, said system comprising:
A. an implantable analyte sensor (324) configured to detect the analyte within the living organism and comprising:
(1) indicator elements (326) configured to exhibit a detectable property based on the amount or concentration of the analyte in proximity to the indicator elements;
(2) sensor elements (328) configured to generate a data signal based on the detectable property exhibited by said indicator elements; and
(3) an interface device (330) configured to receive signals and generate power for powering said sensor elements and to convey data signals generated by said sensor elements; and
B. a transceiver (100) according to any of claims 1 to 5, the interface device (216) configured to convey the power signal to said interface device of said implantable analyte sensor and to receive data signals conveyed by said interface device of said implantable analyte sensor;
and the processor (332) configured to calculate the analyte concentration value based on one or more of the received data signals from the implantable analyte sensor,
wherein the interface device of the implantable analyte sensor is an antenna configured to wirelessly receive the power signal from the transceiver and to wirelessly convey the data signals generated by said sensor elements.

7. The system of claim 6, wherein the interface device of the implantable analyte sensor is an antenna configured to wirelessly receive signals from the transceiver.

## Patentansprüche

1. Am Körper tragbarer Transceiver (100) zum Verbinden mit einem implantierbaren Analytsensor (324) in einem kontinuierlichen Analytüberwachungssystem, wobei der Transceiver Folgendes umfasst:
eine Schnittstellenvorrichtung (216), die dazu konfiguriert ist, ein Leistungssignal an den implantierbaren Analytsensor zu übermitteln und Datensignale von dem implantierbaren Analytsensor zu empfangen;
eine Batterie (212);
einen Prozessor (332), der dazu konfiguriert ist, einen Analytkonzentrationswert basierend auf einem oder mehreren der empfangenen Datensignale von dem implantierbaren Analytsensor zu berechnen;
eine Benachrichtigungsvorrichtung (342), die einen Vibrationsmotor (102) enthält, wobei die Benachrichtigungsvorrichtung dazu konfiguriert ist, Folgendes zu erzeugen:
ein Vibrationssignal mit einem ersten Muster, um anzugeben, dass der berechnete Analytkonzentrationswert einen Schwellenwert überschritten hat oder unterschritten hat, und
ein Vibrationssignal mit einem zweiten Muster, das sich von dem ersten Muster unterscheidet, um anzugeben, dass ein Leistungspegel der Batterie unter einem Schwellenwert liegt; und
eine Kommunikationsschaltung (336), die dazu konfiguriert ist, Daten an eine externe Vorrichtung (338, 340) zu senden,
wobei die Schnittstellenvorrichtung eine Antenne ist, die dazu konfiguriert ist, das Leistungssignal drahtlos an eine Antenne (330) des implantierbaren Analytsensors zu übermitteln und die Datensignale drahtlos von der Antenne des implantierbaren Analytsensors zu empfangen.

2. Transceiver nach Anspruch 1, wobei die Benachrichtigungsvorrichtung dazu konfiguriert ist, ein oder mehrere schwingende, akustische und visuelle Signale zu erzeugen, wenn der Transceiver zum optimalen Signalaustausch zwischen dem implantierbaren Analytsensor und dem Transceiver platziert ist.

3. Transceiver nach Anspruch 1, wobei die Benachrichtigungsvorrichtung eine oder mehrere LEDs umfasst, die dazu konfiguriert sind, ein oder mehrere visuelle Signale zu erzeugen.

4. Transceiver nach Anspruch 1, umfassend mindestens eines der folgenden Merkmale:
(i) einem Beschleunigungsmesser;
(ii) einen in den Beschleunigungsmesser eingebetteten Temperatursensor;
(iii) eine Global Positioning System-, GPS-, Einheit, die dazu konfiguriert ist, ein GPS-Signal zu erfassen und Standortmessungen vorzunehmen; und
(iv) einen Temperatursensor.

5. Transceiver nach Anspruch 1, der dazu konfiguriert ist, durch ein Band und/oder einen Klebstoff am lebenden tierischen oder menschlichen Körper festgehalten zu werden.

6. System (300) zum Nachweisen einer Menge oder Konzentration eines Analyten in vivo innerhalb eines lebenden Organismus, wobei das System Folgendes umfasst:
A. einen implantierbaren Analytsensor (324), der dazu konfiguriert ist, den Analyten innerhalb des lebenden Organismus nachzuweisen, und Folgendes umfasst:
(1) Indikatorelemente (326), die dazu konfiguriert sind, eine nachweisbare Eigenschaft basierend auf der Menge oder Konzentration des Analyten in der Nähe der Indikatorelemente aufzuweisen;
(2) Sensorelemente (328), die dazu konfiguriert sind, ein Datensignal basierend auf der nachweisbaren Eigenschaft zu erzeugen, die von den Indikatorelementen aufgewiesen wird; und
(3) eine Schnittstellenvorrichtung (330), die dazu konfiguriert ist, Signale zu empfangen und Energie zu erzeugen, um die Sensorelemente mit Energie zu versorgen und von den Sensorelementen erzeugte Datensignale zu übermitteln; und
B. einen Transceiver (100) nach einem der Ansprüche 1 bis 5, wobei die Schnittstellenvorrichtung (216) dazu konfiguriert ist, das Leistungssignal an die Schnittstellenvorrichtung des implantierbaren Analytsensors zu übermitteln und Datensignale zu empfangen, die von der Schnittstellenvorrichtung des implantierbaren Analytsensors übermittelt werden;
und der Prozessor (332) dazu konfiguriert ist, den Analytkonzentrationswert basierend auf einem oder mehreren der empfangenen Datensignale von dem implantierbaren Analytsensor zu berechnen, wobei die Schnittstellenvorrichtung des implantierbaren Analytsensors eine Antenne ist, die dazu konfiguriert ist, das Leistungssignal drahtlos von dem Transceiver zu empfangen und die von den Sensorelementen erzeugten Datensignale drahtlos zu übermitteln.

7. System nach Anspruch 6, wobei die Schnittstellenvorrichtung des implantierbaren Analytsensors eine Antenne ist, die dazu konfiguriert ist, drahtlos Signale von dem Transceiver zu empfangen.

## Revendications

1. Émetteur-récepteur portable sur le corps (100) destiné à s'interfacer avec un capteur d'analyte implantable (324) dans un système de surveillance continue d'analyte, l'émetteur-récepteur comprenant :
un dispositif d'interface (216) configuré pour transférer un signal de puissance au capteur d'analyte implantable et pour recevoir des signaux de données en provenance du capteur d'analyte implantable ;
une batterie (212) ;
un processeur (332) configuré pour calculer une valeur de concentration d'analyte sur la base d'un ou plusieurs des signaux de données reçus en provenance du capteur d'analyte implantable ;
un dispositif de notification (342) incluant un moteur de vibration (102), dans lequel le dispositif de notification est configuré pour générer :
un signal vibratoire ayant un premier motif pour indiquer que la valeur de concentration d'analyte calculée a dépassé ou est tombée en dessous d'une valeur de seuil, et
un signal vibratoire ayant un second motif différent du premier motif pour indiquer qu'un niveau de puissance de la batterie est inférieur à un seuil ; et
un circuit de communication (336) configuré pour transmettre des données à un dispositif externe (338, 340),
dans lequel le dispositif d'interface est une antenne configurée pour transférer sans fil le signal de puissance à une antenne (330) du capteur d'analyte implantable et pour recevoir sans fil les signaux de données en provenance de l'antenne du capteur d'analyte implantable.

2. Emetteur-récepteur selon la revendication 1, dans lequel le dispositif de notification est configuré pour générer un ou plusieurs parmi un signal vibratoire, auditif et visuel si l'émetteur-récepteur est placé pour un échange de signal optimal entre le capteur d'analyte implantable et l'émetteur-récepteur.

3. Émetteur-récepteur selon la revendication 1, dans lequel le dispositif de notification comprend une ou plusieurs LED configurées pour générer un ou plusieurs signaux visuels.

4. Emetteur-récepteur selon la revendication 1, comprenant au moins l'une des fonctions suivantes :
(i) un accéléromètre ;
(ii) un capteur de température intégré dans l'accéléromètre ;
(iii) une unité de système de positionnement global, GPS, configurée pour acquérir un signal GPS et prendre des mesures de localisation ; et
(iv) un capteur de température.

5. Emetteur-récepteur selon la revendication 1, configuré pour être maintenu en place sur l'animal vivant ou le corps humain par une bande et/ou un adhésif.

6. Système (300) pour détecter une quantité ou concentration d'un analyte in vivo dans un organisme vivant, ledit système comprenant :
A. un capteur d'analyte implantable (324) configuré pour détecter l'analyte dans l'organisme vivant et comprenant :
(1) des éléments indicateurs (326) configurés pour présenter une propriété détectable sur la base de la quantité ou la concentration de l'analyte à proximité des éléments indicateurs ;
(2) des éléments capteurs (328) configurés pour générer un signal de données sur la base de la propriété détectable présentée par lesdits éléments indicateurs ; et
(3) un dispositif d'interface (330) configuré pour recevoir des signaux et générer de la puissance pour alimenter lesdits éléments capteurs et pour transférer des signaux de données générés par lesdits éléments capteurs ; et
B. un émetteur-récepteur (100) selon l'une quelconque des revendications 1 à 5, le dispositif d'interface (216) étant configuré pour transférer le signal de puissance vers ledit dispositif d'interface dudit capteur d'analyte implantable et pour recevoir des signaux de données transférés par ledit dispositif d'interface dudit capteur d'analyte implantable ;
et le processeur (332) configuré pour calculer la valeur de concentration d'analyte sur la base d'un ou plusieurs des signaux de données reçus en provenance u capteur d'analyte implantable,
dans lequel le dispositif d'interface du capteur d'analyte implantable est une antenne configurée pour recevoir sans fil le signal de puissance en provenance de l'émetteur-récepteur et pour transférer sans filles signaux de données générés par lesdits éléments capteurs.

7. Système selon la revendication 6, dans lequel le dispositif d'interface du capteur d'analyte implantable est une antenne configurée pour recevoir sans fil des signaux en provenance de l'émetteur-récepteur.
